# EUROPEAN PATENT SPECIFICATION

(11) **EP 2 878 327 B1**
(45) Date of publication and mention of the grant of the patent: **29.06.2016**
(21) Application number: 13822661.8
(22) Date of filing: 24.07.2013
(51) Int. Cl.: A61M 15/00, A61M 11/02, B01D 63/06

(54) **APPARATUS FOR INHALATION OF MEDICINE**
VORRICHTUNG ZUR INHALATION VON ARZNEIMITTELN
APPAREIL POUR INHALATION DE MÉDICAMENT

(30) Priority: 24.07.2012 KR 20120080788
(43) Date of publication of application: 03.06.2015
(73) Proprietor: Korea Research Institute of Chemical Technology, Daejeon 305-343 (KR)
(72) Inventor: LEE, Kyu Hong, Jeongeup-si Jeollabuk-do 580-185 (KR); HEO, Yong Ju, Gwangju 500-762 (KR)
(74) Representative: v. Bezold & Partner Patentanwälte - PartG mbB
(86) International application number: PCT/KR2013/006629
(87) International publication number: WO 2014/017827

(56) References cited:
- EP-A1- 1 712 220
- EP-A2- 0 166 476
- JP-A- 2007 097 830
- KR-A- 20010 101 991
- KR-A- 20070 073 865
- KR-A- 20090 114 436
- US-A1- 2005 196 345

## Description

### TECHNICAL FIELD

The present invention relates to a drug inhalation device, and more particularly, to a drug inhalation device **in which** drug particles in an aqueous solution, in a liquid aerosol form, are converted into dry drug particles by removing moisture therefrom by means of an evaporation unit so as to be inhaled by means of an inhalation mechanism, so that the drug particles can penetrate deep into the lungs of a patient along the respiratory track and thus can be applied to the treatment of pulmonary diseases or a variety of other treatments using the same, and in which the evaporation unit is configured in such a manner as to supply dry air using an air compressor used in an aerosol unit, so that the necessity for a separate device is eliminated, thereby achieving configuration simplification thereof, facilitating the manufacture thereof, and reducing the manufacturing cost.

### BACKGROUND ART

In general a drug inhalation device is a device which enables a patient to inhale a drug through his or her respiratory track. The drug inhalation device is widely used for the treatment of disease such as asthma, pulmonary disease or the like. Recently, the drug inhalation device is used as a device for injecting a variety of drugs into a patient's nose or longs.

The drug inhalation device is configured such that an aqueous drug solution in which drug particles are dissolved in water is converted into fine particles through aerosolization, and the drug aerosol particles are inhaled into the patient's oral cavity or nasal cavity through a separate inhalation mechanism.

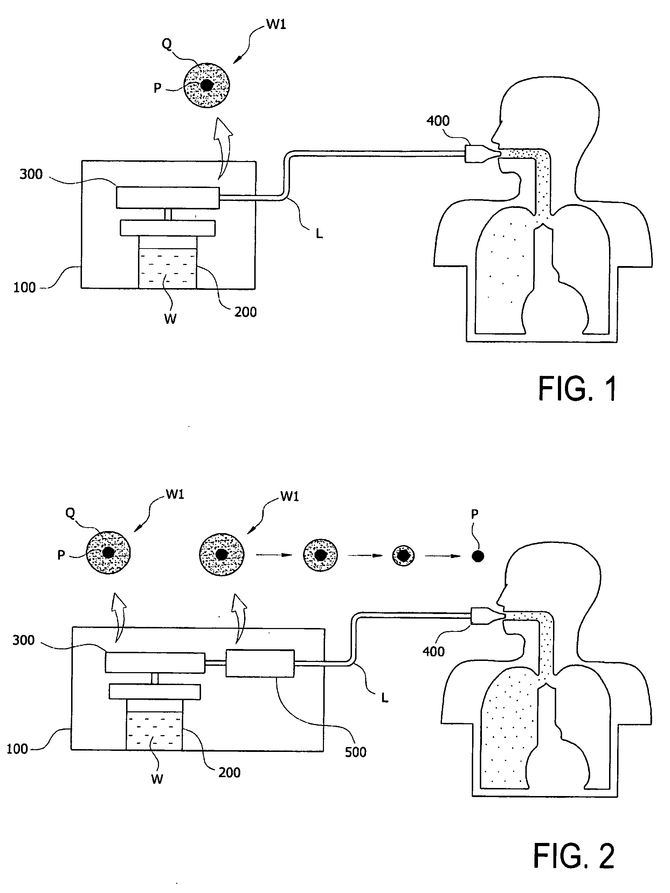

FIG. 1 is a conceptual view illustrating a configuration of a general drug inhalation device according to the prior art.

As shown in FIG. 1, the general drug inhalation device according to the prior art includes a drug solution storage unit 200 that is disposed inside a main body 100 and configured to store an aqueous drug solution therein, and an aerosol unit 300 that is disposed inside a main body 100 and configured to aerosolize the aqueous drug solution W stored in the drug solution storage unit 200. The aerosol unit 300 can aerosolize the aqueous drug solution W in such a manner as to supply separate compressed air, and may b e configured in various manners such as a supersonic method, a jet injection method and the like. A separate inhalation line L is connected to the aerosol unit 300 and the inhalation mechanism 400 such as a mask or a mouthpiece is coupled to one end of the inhalation line L so as to be disposed at a user's respiratory organ.

By virtue of this configuration, the drug particles W1 in an aqueous solution are converted into an aerosol form and are inhaled into the patient's respiratory organ through the inhalation mechanism 400 while passing through the inhalation line L during the respiration of the patient. As such, the drug particles W1 inhaled into the patient's respiratory organ are directly delivered to the patient's bronchus or lungs along his or her respiratory track.

However, the conventional general drug inhalation device according to the prior art entails a problem in that since the drug particles W1 in an aqueous solution, in an aerosol form, which are generated from the aerosol unit 300 are present in a liquid form, the drug particles W1 are nearly absorbed in the patient's oral cavity or throat but do not penetrate deep into the lungs along his or her respiratory track as shown in FIG. 1.

In other words, since the drug particles W1 in an aqueous solution, in a liquid aerosol form, which are generated from the aerosol unit 300 are present in the form in which water particles Q surround the outer spaces of the dry drug particles P as shown in FIG. 1, they are large in size and heavyweight, and thus the drug particles W1 are nearly absorbed in the patient's oral cavity or throat in the process where the drug particles W1 are inhaled into or her respiratory organ through the inhalation mechanism 400 but do not penetrate deep into the lungs along his or her respiratory track.

Therefore, the conventional drug inhalation device involves a problem in that since the drug does not penetrate deep into a patient's lungs, the device is used merely for the purpose of the treatment of a bronchus region and has a limitation in use for the purpose of treating pulmonary diseases. In addition, in the case where the drug particles are required to be inhaled deep into the lungs along the patient's respiratory track, the drug penetrates inhaled into the lungs in a relatively small amount, resulting in an increase in the amount of the drug used.
EP 0 166 476 A2 discloses an aerosol generator-inhalator, including a nebulizer and an inhalating device as well as an aerosol-drying chamber and a device for supplying drying air being arranged between the nebulizer and the inhalating device. A compressed air inhaler for pulmonary application of powder aerosols is disclosed in US 2005/0196345 A1, which comprises a nozzle body with a compressed air supply channel and a liquid supply channel, a drying chamber and an aerosol exit.

### DISCLOSURE OF INVENTION

### TECHNICAL PROBLEM

Accordingly, the present invention has been made to solve the above-mentioned problems occurring in the prior art, and it is an object of the present invention to provide a drug inhalation device, and more particularly, to a drug inhalation device in which drug particles in an aqueous solution, in a liquid aerosol form, are converted into dry drug particles by removing moisture therefrom by means of an evaporation unit so as to be inhaled by means of an inhalation mechanism, so that the drug particles can penetrate deep into the lungs of a patient along the respiratory track and thus can be applied to the treatment of pulmonary diseases or a variety of other treatments using the same.

Another object of the present invention is to provide a drug inhalation device in which an evaporation unit for removing moisture from drug particles in an aqueous solution is configured in such a manner as to supply dry air using an air compressor used in an aerosol unit, so that the necessity for a separate device is eliminated, thereby achieving configuration simplification thereof, facilitating the manufacture thereof, and reducing the manufacturing cost.

Still another object of the present invention is to provide a drug inhalation device which is configured such that it additionally includes a heating module besides a dry air supply module to further accelerate the evaporation of drug particles in an aqueous solution, so that dry drug particles in a highly dry state can be produced.

### TECHNICAL SOLUTION

To achieve the above objects, the present invention provides a drug inhalation device including: a main body having disposed therein a drug solution storage unit configured to store an aqueous drug solution therein; an aerosol unit configured to aerosolize the aqueous drug solution stored in the drug solution storage unit; an inhalation mechanism connected to the aerosol unit and mounted at a user's respiratory organ; and an evaporation unit configured to be supplied with drug particles in an aqueous solution, in a liquid aerosol form, which is generated from the aerosol unit, remove moisture from the drug particles, and convert the moisture-removed drug particles into dry drug particles, wherein the evaporation unit includes: an evaporation casing having an evaporation chamber defined in an internal space thereof; a particle flowing pipe configured to penetrate through the evaporation casing so as to pass through the evaporation chamber, the particle flowing pipe being connected at both ends thereof to the aerosol unit and inhalation mechanism; and a dry air supply module configured to supply dry air to the evaporation chamber, wherein the particle flowing pipe is formed as a breathable membrane that allows air and vapor to pass therethrough and does not allow the dry drug particles to pass therethrough, and moisture is removed from the drug particles in the aqueous solution, in a liquid aerosol form, that is introduced into the particle flowing pipe from the aerosol unit in a process in which the drug particles pass through the particle flowing pipe so that the drug particles are inhaled, in a dry particle form, into the user's respiratory organ through the inhalation mechanism.

In this case, the evaporation casing may include an inlet port and an outlet port formed at both sides thereof so as to allow dry air to flow in and out of the evaporation chamber therethrough, and the dry air supply module may include an air compressor that is connected to the inlet port and configured to supply compressed dry air.

In addition, the particle flowing pipe may be arranged in a zig-zag pattern in the evaporation chamber.

In addition, the particle flowing pipe may be arranged in a branched pattern in the evaporation chamber.

In addition, the evaporation unit may further include a heating module configured to heat the internal space of the evaporation casing.

In addition, the heating module may be configured to surround the outer peripheral surface of the evaporation casing.

### ADVANTAGEOUS EFFECTS

The drug inhalation device according to the embodiments of the present invention as constructed above have the following advantageous effects. First, drug particles in an aqueous solution, in a liquid aerosol form, are converted into dry drug particles by removing moisture therefrom by means of an evaporation unit so as to be inhaled by means of an inhalation mechanism, so that the drug particles can penetrate deep into the lungs of a patient along the respiratory track and thus can be applied to the treatment of pulmonary diseases or a variety of other treatments using the same. Thus, the applicable range of the drug inhalation device can be extended and a loss of the drug particles does not occur, thereby reducing the amount of drugs used.

In addition, an evaporation unit for removing moisture from drug particles in an aqueous solution is configured in such a manner as to supply dry air using an air compressor used in an aerosol unit, so that the necessity for a separate device is eliminated, thereby achieving configuration simplification thereof, facilitating the manufacture thereof, and reducing the manufacturing cost.

Moreover, the drug inhalation device is configured such that it additionally includes a heating module besides a dry air supply module to further accelerate the evaporation of drug particles in an aqueous solution, so that dry drug particles in a highly dry state can be produced.

### BRIEF DESCRIPTION OF THE DRAWINGS

FIG. 1 is a conceptual view illustrating a configuration of a general drug inhalation device according to the prior art;
FIG. 2 is a conceptual view illustrating a configuration of a drug inhalation device according to an embodiment of the present invention;
FIG. 3 is a schematic view illustrating a configuration of an evaporation unit of a drug inhalation device according to an embodiment of the present invention;
FIG. 4 is a schematic view illustrating various modifications of a particle flowing pipe of an evaporation unit according to an embodiment of the present invention; and
FIG. 5 is a schematic view illustrating another modification of an evaporation unit of a drug inhalation device according to an embodiment of the present invention.

### BEST MODE FOR CARRYING OUT THE INVENTION

Now, preferred embodiments of the present invention will be described hereinafter in detail with reference to the accompanying drawings. It should be noted that the same elements in the drawings are denoted by the same reference numerals although shown in different figures. In the following description, the detailed description on known function and constructions unnecessarily obscuring the subject matter of the present invention will be avoided hereinafter.

FIG. 2 is a conceptual view illustrating a configuration of a drug inhalation device according to an embodiment of the present invention.

A drug inhalation device according to an embodiment of the present invention is a device that enables a patient to inhale a drug through his or her respiratory organ. The drug inhalation device includes a main body 100, having an accommodating space defined therein, a drug solution storage unit 200 that is disposed inside the main body 100 and configured to store an aqueous drug solution therein, an aerosol unit 300 configured to aerosolize the aqueous drug solution W stored in the drug solution storage unit 200, an inhalation mechanism 400 connected to the aerosol unit 300 and mounted at a user's respiratory organ, and an evaporation unit 500 disposed between the aerosol unit 300 and the inhalation mechanism 400.

The main body 100 is formed in the shape of a casing having an accommodating space defined therein. The main body 100 may be used both in a stationary form in which it is placed at a specific position in a hospital or at home, and in a portable form in which a user can grip it his or her hands. The drug solution storage unit 200 and the aerosol unit 300 are disposed inside the main body 100. The drug solution storage unit 200 and the aerosol unit 300 are mounted inside the main body 100 so as to have a proper size depending on whether the main body 100 is configured in the stationary form or in the portable form.

The drug solution storage unit 200 stores an aqueous drug solution W in a state in which drug particles are dissolved in water. The aerosol unit 300 is configured to aerosolize the aqueous drug solution W stored in the drug solution storage unit 200. The aerosolization of the aqueous drug solution W by the aerosol unit 300 may be performed by either a method of supplying compressed air through a separate air compressor or a supersonic method employing a supersonic vibrator. This configuration of the aerosol unit 300 is used as a known technique, and thus a detailed description thereof will be omitted to avoid redundancy.

The inhalation mechanism 400 is connected to the aerosol unit 300 through an inhalation line L, and the inhalation mechanism 400 such as a mask or a mouthpiece is coupled to one end of the inhalation line L so as to be disposed at the user's respiratory organ. In this case, the evaporation unit 500 is disposed between the aerosol unit 300 and the inhalation mechanism 400, and is connected to the aerosol unit 300 and the inhalation mechanism 400 through the inhalation line L.

The evaporation unit 500 is configured to be supplied with drug particles W1 in an aqueous solution, in a liquid aerosol form, which are generated from the aerosol unit 300, remove moisture from the drug particles W1, and convert the moisture-removed drug particles W1 into dry drug particles. The evaporation unit 500 can be disposed inside the main body 100 as shown in FIG. 2, but may be separately disposed outside the main body 100.

By virtue of this configuration, the drug particles W1 in an aqueous solution, in a liquid aerosol form, which are generated from the aerosol unit 300 are converted into the dry drug particles P in which moisture has been removed while passing through the evaporation unit 500, and then are inhaled into the user's respiratory organ through the inhalation mechanism 400.

More specifically, the drug particles W1 in an aqueous solution, in a liquid aerosol form, which are generated from the aerosol unit 300 are present in the form in which water particles Q surround the outer spaces of the dry drug particles P as shown in FIG. 2. The evaporation unit 500 is supplied with the drug particles W1 in an aqueous solution, in an aerosol form, and serves to remove moisture from the drug particles W1. In other words, the evaporation unit 500 functions to evaporate moisture from the liquid aerosol particles, i.e., the drug particles W1 in an aqueous solution and converts the moisture-removed drug particles W1 into dry drug particles P which are solid aerosol particles.

As a result, outer water particles Q are continuously evaporated from the drug particles W1 in an aqueous solution in the process where the drug particles W1 pass through the evaporation unit 500, and the drug particles W1 are finally converted into the dry drug particles P in which the water particles Q have been all removed as shown in FIG. 2. In this case, the dry drug particles P become relatively small in size as compared to the drug particles W1 in an aqueous solution. Generally, the drug particles W1 in an aqueous solution have a particle size of a micro unit, and the dry drug particles have a particle size of a nano unit. As such, the dry drug particles P converted by the evaporation unit 500 are deliverd to the inhalation mechanism 400 through the inhalation line L, and thus are inhaled into the user's respiratory organ.

Thus, the drug inhalation device according to an embodiment of the present invention enables the drug particles W1 in an aqueous solution to be converted into the dry drug particles P by means of the evaporation unit 500, and then enables the converted dry drug particles P to be inhaled into the user's respiratory organ through the inhalation mechanism 400, so that the dry drug particles P can penetrate deep into the lungs of a patient unlike the prior art technique.

In other words, since the drug particles W1 in an aqueous solution, in a liquid aerosol form are relatively large in size and weight as mentioned above in the prior art, they are nearly absorbed in the patient's oral cavity or throat but do not penetrate deep into the lungs along his or her respiratory track in the process where the drug particles W1 are inhaled into the patient's respiratory organ through the inhalation mechanism 400. On the other hand, since the moisture-removed dry drug particles P in a solid aerosol form have a particle size of a nano unit and are lightweight, they can penetrate deep into the patient's lungs during the respiration.

Thus, the drug inhalation device according to an embodiment of the present invention can be used for the treatment of bronchus and pulmonary diseases. The drug inhalation device can be utilized in various types of treatments such as a method of penetrating drug particles deep into the patient's lungs to circulate blood.

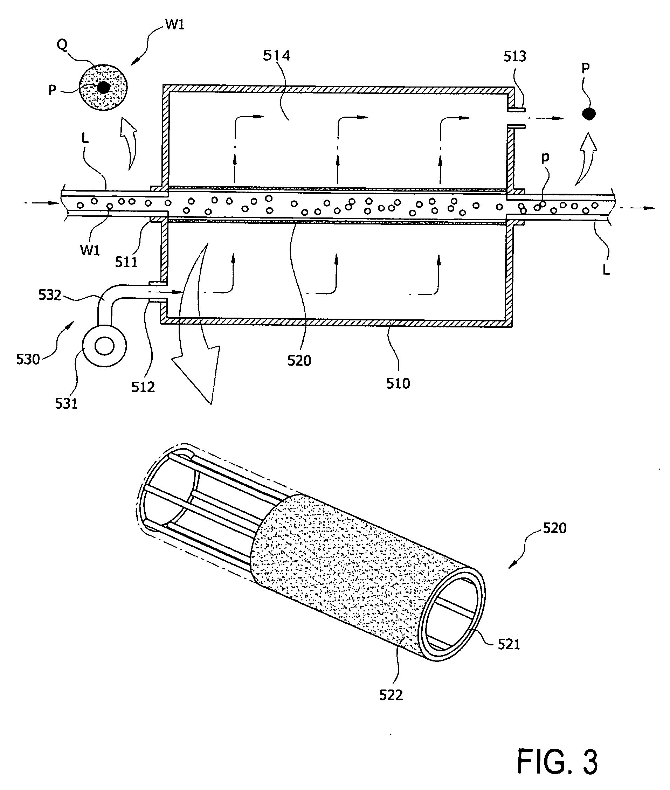

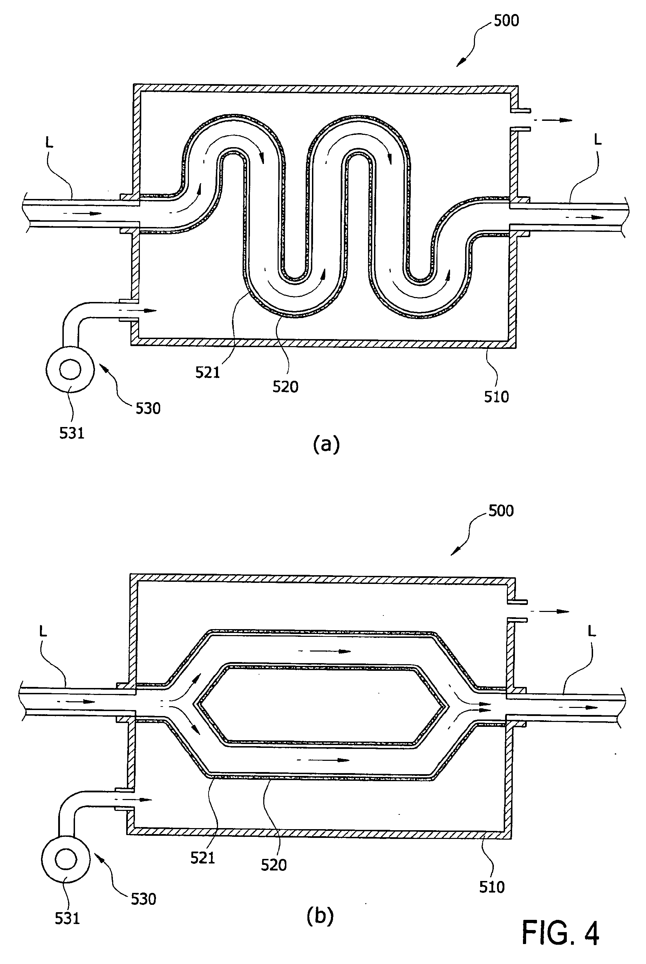

FIG. 3 is a schematic view illustrating a configuration of an evaporation unit of a drug inhalation device according to an embodiment of the present invention, and FIG. 4 is a schematic view illustrating various modifications of a particle flowing pipe of an evaporation unit according to an embodiment of the present invention.

The evaporation unit 500 of the drug inhalation device according to an embodiment of the present invention an evaporation casing 510 having an evaporation chamber 514 defined in an internal space thereof; a particle flowing pipe 520 configured to penetrate through the evaporation casing 510 so as to pass through the evaporation chamber 514; and a dry air supply module 530 configured to supply dry air to the evaporation chamber 514 as shown in FIG. 3.

The evaporation casing 510 has a connection port 511 formed respectively at both sides thereof so as to allow the inhalation line L to be inserted thereto so that the evaporation casing 510 is connected to the aerosol unit 300 and the inhalation mechanism 400 through the connection ports 511. The evaporation casing 510 includes an inlet port 512 formed at one side thereof so as to allow dry air to flow in the evaporation chamber 514 from the dry air supply module 530 therethrough, and an outlet port 513 formed at the other side thereof so as to allow the dry air introduced into the evaporation chamber 514 to flow out of the evaporation chamber 514 therethrough.

The particle flowing pipe 520 is disposed inside the evaporation casing 510 so as to fluidically communicate with the connection port 511 so that the particle flowing pipe 520 is connected at both ends thereof to the aerosol unit 300 and the inhalation mechanism 400 through the inhalation line L. Thus, the drug particles W1 in an aqueous solution, in a liquid aerosol form, which are generated from the aerosol unit 300 is introduced into the particle flowing pipe 520 through the inhalation line L, and then flows toward the inhalation mechanism 400 along the inhalation line L.

The particle flowing pipe 520 is formed as a breathable membrane 522 that allows air and vapor to pass therethrough and does not allow the dry drug particles P to pass therethrough. In this case, the particle flowing pipe 520 formed as the breathable membrane 522 includes a frame 521 of a pipe shape mounted therein so as to implement a pipe structure.

In other words, the particle flowing pipe 520 includes the frame 521 of a cylindrical pipe shape and the breathable membrane 522 that encircles the outer surface of the frame 521 as shown in FIG. 3. In this case, the frame 521 may be configured in various shapes. For example, the frame 521 may be formed as a structure having a cylindrical pipe shape or a simple metal mesh shape. In addition, the breathable membrane 522 is a membrane that performs a selective permeation function, and is configured to allow air and vapor to pass therethrough and not to allow the dry drug particles P to pass therethrough. For example, a Nafion membrane may be used as the breathable membrane 522.

The dry air supply module 530 is configured to supply dry air to the evaporation chamber 514. As shown in FIG. 3, **the** dry air supply module 530 may include an air compressor 531 that compresses air and supplies the compressed dry air to the evaporation chamber 514, and an air supply line 532 that is connected to the air compressor 531. The dry air supply module 530 is configured such that the air supply line 532 is coupled to the inlet port 512 of the evaporation casing 510 so that the compressed dry air from the air compressor 531 is supplied to the evaporation chamber 514 through the air supply line 532. In this case, the air compressor 531 may be separately configured, but in the case where the aerosol unit 300 is configured in such a manner as to supply the compressed air through the air compressor, the compressed dry air may be supplied to the evaporation chamber 514 using the air compressor used in the aerosol unit 300 without a separate air compressor.

As the dry air is supplied to the evaporation chamber 514 of the evaporation casing 510 by means of the dry air supply module 530, the internal space of the evaporation chamber 514 becomes a state in which the relative humidity is very low. In the meantime, the internal space of the particle flowing pipe 520 becomes a state in which the relative humidity is relatively high due to evaporation of moisture from the drug particles W1 in an aqueous solution. In this case, since the particle flowing pipe 520 is formed as the breathable membrane 522 that allows air and vapor to pass therethrough, the internal space of the evaporation chamber 514 fluidically communicate with and the internal space of the particle flowing pipe 520. Thus, the water particles evaporated in the internal space of the particle flowing pipe 520 are diffused to the evaporation chamber 514 while passing through the breathable membrane 522 so that the internal space of the particle flowing pipe 520 also becomes a state in which the relative humidity is low. In other words, the internal space of the particle flowing pipe 520 and the internal space of the evaporation chamber 514 are maintained in a state in which the relative humidity is low by the dry air supplied being to the inside of the evaporation chamber 514, and thus the drug particles W1 in an aqueous solution are more actively evaporated in the internal space of the particle flowing pipe 520.

Accordingly, the drug particles W1 in an aqueous solution, in a liquid aerosol form, which are generated from the aerosol unit 300 are introduced into the particle flowing pipe 520 of the evaporation unit 500 through the inhalation line L, and then the evaporation of the drug particles W1 occurs actively according to the surrounding state in which the relative humidity is low in the process where the drug particles W1 pass through the particle flowing pipe 520. Resultantly, the drug particles W1 are converted into the dry drug particles P in which moisture has been removed. As such, the converted dry drug particles P are supplied to the inhalation mechanism 400 through the inhalation line L, and are inhaled into the user's respiratory organ through the inhalation mechanism 400.

Meanwhile, moisture is removed from the drug particles W1 in an aqueous solution by a phenomenon in which the drug particles W1 are evaporated in the process where the drug particles W1 pass through the particle flowing pipe 520. Thus, preferably, the flow channel of the particle flowing pipe 520 is made long so that the evaporation of the drug particles W1 occurs for a sufficient long period of time.

For example, as shown in FIG. 4(a), the particle flowing pipe 520 is preferably arranged in a zig-zag pattern in the evaporation chamber 514 so that the evaporation time of the drug particles W1 in an aqueous solution, which pass through the particle flowing pipe 520 can be increased, leading to conversion of the drug particles W1 into the dry drug particles P in a more highly dry state. In addition, as shown in FIG. 4(b), the particle flowing pipe 520 is arranged in a branched pattern, i.e., arranged to be branched into two pipes in the evaporation chamber 514 so that the drug particles W1 in an aqueous solution are evaporated in a dispersed state while passing through the branched particle flowing pipe 520, leading to conversion of the drug particles W1 into the dry drug particles P in a more highly dry state. In this case, the particle flowing pipe 520 is branched into two pipes, but may be branched into two or more pipes.

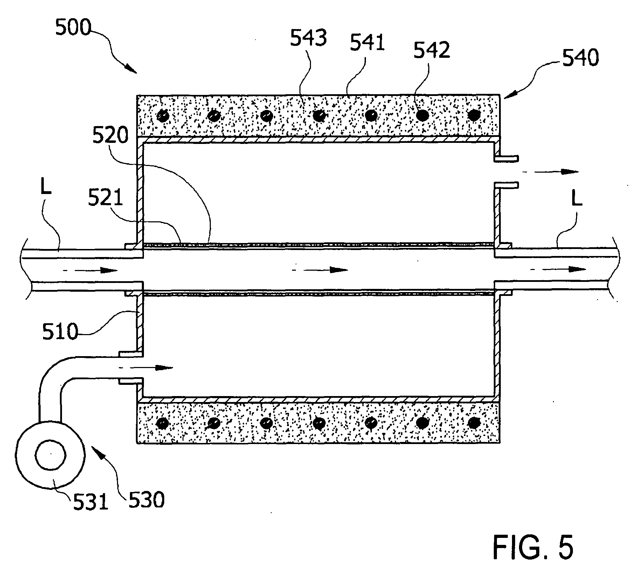

FIG. 5 is a schematic view illustrating another modification of an evaporation unit of a drug inhalation device according to an embodiment of the present invention.

The evaporation unit 500 of the drug inhalation device according to an embodiment of the present invention may further include a separate heating module 540 that can heat the evaporation chamber 514 of the evaporation casing 510.

In other words, if the relative humidity of the evaporation chamber 514 is caused to be low, the evaporation of moisture from the drug particles W1 passing through the particle flowing pipe 520 is further accelerated. As described above, in addition to a method in which dry air is supplied to the evaporation chamber 514 by means of the dry air supply module 530, the temperature of the evaporation chamber 514 is risen by the heating module 540 so that the relative humidity of the evaporation chamber 514 can be further reduced, and thus the evaporation of moisture from the drug particles W1 in an aqueous solution can be further accelerated.

The heating module 540 is configured to surround the outer peripheral surface of the evaporation casing 510 so that the evaporation chamber 514 is heated to lower the total relative humidity of the internal spaces of the evaporation chamber 514 and the particle flowing pipe 520 as shown in FIG. 5a. Alternatively, the heating module 540 may be disposed inside the evaporation chamber 514 so as to surround the outer peripheral surface of the particle flowing pipe 520.

The eating module 540 may be configured in various shapes, but may include a heating cover 541 made of a fabric material having flexibility, a heating coil 542 disposed in the internal space of the heating cover 541, and a filling material 543 disposed in the internal space of the heating cover 541 to transfer heat of the heating coil as shown in FIG. 5. Herein, the heating coil 542 may be configured to be supplied with electric power to generate heat. By virtue of this configuration, the heating module 540 has flexibility and can surround the evaporation casing 510 so that a heat loss can be minimized. Of course, the heating module 540 may be implemented in various manners to exhibit a heat-generating function such as chemical-thermal decomposition.

While the present invention has been described in connection with the exemplary embodiments illustrated in the drawings, they are merely illustrative and the invention is not limited to these embodiments. It will be appreciated by a person having an ordinary skill in the art that various equivalent modifications and variations of the embodiments can be made without departing from the scope of the present invention. Therefore, the true technical scope of the present invention should be defined by the technical spirit of the appended claims.

## Claims

1. A drug inhalation device comprising: a main body (100) having disposed therein a drug solution storage unit (200) configured to store an aqueous drug solution therein; an aerosol unit (300) configured to aerosolize the aqueous drug solution stored in the drug solution storage unit (200); an inhalation mechanism (400) connected to the aerosol unit (300) and configured to be mounted at a user's respiratory organ; and an evaporation unit (500) configured to be supplied with drug particles in an aqueous solution, in a liquid aerosol form, which is generated from the aerosol unit (300), remove moisture from the drug particles, and convert the moisture-removed drug particles into dry drug particles, wherein the evaporation unit (500) comprises:
an evaporation casing (510) having an evaporation chamber (514) defined in an internal space thereof; and
a dry air supply module (530) configured to supply dry air to the evaporation chamber (514),
**characterized by**
a particle flowing pipe (520) configured to penetrate through the evaporation casing (510) so as to pass through the evaporation chamber (514), the particle flowing pipe (520) being connected at both ends thereof to the aerosol unit (300) and inhalation mechanism (400); wherein
the particle flowing pipe (520) is formed as a breathable membrane (522) that allows air and vapor to pass therethrough and does not allow the dry drug particles to pass therethrough, and moisture is removed from the drug particles in the aqueous solution, in a liquid aerosol form, that is introduced into the particle flowing pipe (520) from the aerosol unit (300) in a process in which the drug particles pass through the particle flowing pipe (520) so that the drug particles can be inhaled, in a dry particle form, into the user's respiratory organ through the inhalation mechanism (400).

2. The drug inhalation device according to claim 1, wherein the evaporation casing (510) comprises an inlet port (512) and an outlet port (513) formed at both sides thereof so as to allow dry air to flow in and out of the evaporation chamber (514) therethrough, and the dry air supply module (530) comprises an air compressor that is connected to the inlet port (512) and configured to supply compressed dry air.

3. The drug inhalation device according to claim 2, wherein the particle flowing pipe (520) is arranged in a zig-zag pattern in the evaporation chamber (514).

4. The drug inhalation device according to claim 2, wherein the particle flowing pipe (520) is arranged in a branched pattern in the evaporation chamber (514).

5. The drug inhalation device according to any of claim 1 to claim 4, wherein the evaporation unit (500) further comprises a heating module (540) configured to heat the internal space of the evaporation casing (510).

6. The drug inhalation device according to claim 5, wherein the heating module (540) is configured to surround the outer peripheral surface of the evaporation casing (510).

## Patentansprüche

1. Arzneimittelinhalationsvorrichtung, umfassend: einen Hauptkörper (100), der eine darin angeordnete Arzneimittellösungsspeichereinheit (200) aufweist, die konfiguriert ist, in dieser eine wässrige Arzneimittellösung zu speichern; eine Aerosoleinheit (300), die konfiguriert ist, die wässrige Arzneimittellösung zu versprühen, die in der Arzneimittellösungsspeichereinheit (200) gespeichert ist; einen Inhalationsmechanismus (400), der mit der Aerosoleinheit (300) verbunden und konfiguriert ist, an einem Atemorgan eines Nutzers angebracht zu werden; und eine Verdampfungseinheit (500), die konfiguriert ist, mit Arzneimittelpartikeln in einer wässrigen Lösung, in einer flüssigen Aerosolform, beliefert zu werden, die von der Aerosoleinheit (300) erzeugt wird, Feuchtigkeit von den Arzneimittelpartikeln zu entfernen und die Feuchtigkeits-entfernten Arzneimittelpartikel in trockene Arzneimittelpartikel zu konvertieren, wobei die Verdampfungseinheit (500) umfasst:
ein Verdampfungsgehäuse (510), das eine Verdampfungskammer (514) aufweist, die in einem Innenraum von diesem definiert ist; und
ein Trockenluftzufuhrmodul (530), das konfiguriert ist, trockene Luft zu der Verdampfungskammer (514) zuzuführen,
**gekennzeichnet durch**
eine Partikelfließleitung (520), die konfiguriert ist, **durch** das Verdampfungsgehäuse (510) durchzudringen, um so **durch** die Verdampfungskammer (514) hindurchzutreten, wobei die Partikelfließleitung (520) an beiden Enden von dieser mit der Aerosoleinheit (300) und dem Inhalationsmechanismus (400) verbunden ist; wobei
die Partikelfließleitung (520) als eine atmungsaktive Membran (522) gebildet ist, die erlaubt, dass Luft und Dampf **durch** diese hindurchtreten, und die den trockenen Arzneimittelpartikeln nicht erlaubt, **durch** diese hindurchzutreten, und
Feuchtigkeit wird von den Arzneimittelpartikeln in der wässrigen Lösung, in einer flüssigen Aerosolform, die in die Partikelfließleitung (520) von der Aerosoleinheit (300) eingeführt wird, in einem Prozess entfernt, in dem die Arzneimittelpartikel **durch** die Partikelfließleitung (520) hindurchtreten, so dass die Arzneimittelpartikel, in einer trockenen Partikelform, **durch** den Inhalationsmechanismus (400) in das Atemorgan des Nutzers inhaliert werden können.

2. Arzneimittelinhalationsvorrichtung gemäß Anspruch 1, wobei das Verdampfungsgehäuse (510) einen Einlassanschluss (512) und einen Auslassanschluss (513) umfasst, die an beiden Seiten von diesem gebildet sind, um so der trockenen Luft zu erlauben, in die und aus der Verdampfungskammer (514) durchgehend zu fließen, und das Trockenluftzufuhrmodul (530) einen Luftkompressor umfasst, der mit dem Einlassanschluss (512) verbunden und konfiguriert ist, komprimierte trockene Luft zuzuführen.

3. Arzneimittelinhalationsvorrichtung gemäß Anspruch 2, wobei die Partikelfließleitung (520) mit einem Zick-Zack-Muster in der Verdampfungskammer (514) angeordnet ist.

4. Arzneimittelinhalationsvorrichtung gemäß Anspruch 2, wobei die Partikelfließleitung (520) mit einem verzweigten Muster in der Verdampfungskammer (514) angeordnet ist.

5. Arzneimittelinhalationsvorrichtung gemäß einem von Anspruch 1 bis Anspruch 4, wobei die Verdampfungseinheit (500) ferner ein Heizmodul (540) umfasst, das konfiguriert ist, den Innenraum des Verdampfungsgehäuses (510) zu erwärmen.

6. Arzneimittelinhalationsvorrichtung gemäß Anspruch 5, wobei das Heizmodul (540) konfiguriert ist, die äußere Randoberfläche des Verdampfungsgehäuses (510) zu umgeben.

## Revendications

1. Dispositif d'inhalation de médicament comprenant : un corps principal (100) ayant, disposée dans celui-ci, une unité de stockage de solution de médicament (200) configurée pour y stocker une solution de médicament aqueuse ; une unité d'aérosol (300) configurée pour pulvériser par aérosol la solution de médicament aqueuse stockée dans l'unité de stockage de solution de médicament (200) ; un mécanisme d'inhalation (400) connecté à l'unité d'aérosol (300) et configuré pour être monté au niveau d'un organe de respiration d'un utilisateur ; et une unité d'évaporation (500) configurée pour être alimentée en particules de médicament dans une solution aqueuse, sous forme d'aérosol liquide, qui est générée à partir de l'unité d'aérosol (300), pour éliminer l'humidité des particules de médicament et pour convertir les particules de médicament dont l'humidité a été éliminée en particules de médicament sèches, dans lequel l'unité d'évaporation (500) comprend :
un boîtier d'évaporation (510) ayant une chambre d'évaporation (514) définie dans un espace interne de celui-ci ; et
un module d'alimentation en air sec (530) configuré pour acheminer l'air sec jusqu'à la chambre d'évaporation (514),
**caractérisé par**
un tuyau d'écoulement de particules (520) configuré pour pénétrer à travers le boîtier d'évaporation (510) de manière à passer à travers la chambre d'évaporation (514), le tuyau d'écoulement de particules (520) étant connecté par ses deux extrémités à l'unité d'aérosol (300) et au mécanisme d'inhalation (400) ; et dans lequel
le tuyau d'écoulement de particules (520) est formé comme une membrane respirable (522) qui permet à l'air et à la vapeur de passer à travers celle-ci et ne permet pas aux particules de médicament sèches de passer à travers celle-ci, et l'humidité est éliminée des particules de médicament dans la solution aqueuse, sous une forme d'aérosol liquide, qui est introduite dans le tuyau d'écoulement de particules (520) depuis l'unité d'aérosol (300) lors d'un processus dans lequel les particules de médicament passent à travers le tuyau d'écoulement de particules (520) de telle sorte que les particules de médicament peuvent être inhalées, sous une forme de médicament sec, dans l'organe de respiration de l'utilisateur à travers le mécanisme d'inhalation (400).

2. Dispositif d'inhalation de médicament selon la revendication 1, dans lequel le boîtier d'évaporation (510) comprend un orifice d'entrée (512) et un orifice de sortie (513) formés des deux côtés de celui-ci de manière à permettre à de l'air sec de s'écouler à l'intérieur et à l'extérieur de la chambre d'évaporation (514) à travers ceux-ci, et le module d'alimentation en air sec (530) comprend un compresseur d'air qui est connecté à l'orifice d'entrée (512) et configuré pour acheminer de l'air sec comprimé.

3. Dispositif d'inhalation de médicament selon la revendication 2, dans lequel le tuyau d'écoulement de particules (520) est agencé selon un motif en zigzag dans la chambre d'évaporation (514).

4. Dispositif d'inhalation de médicament selon la revendication 2, dans lequel le tuyau d'écoulement de particules (520) est agencé selon un motif de bifurcation dans la chambre d'évaporation (514).

5. Dispositif d'inhalation de médicament selon l'une quelconque de revendication 1 à la revendication 4, dans lequel l'unité d'évaporation (500) comprend en outre un module de chauffage (540) configuré pour chauffer l'espace interne du boîtier d'évaporation (510).

6. Dispositif d'inhalation de médicament selon la revendication 5, dans lequel le module de chauffage (540) est configuré pour entourer la surface périphérique extérieure du boîtier d'évaporation (510).
